# EUROPÄISCHE PATENTSCHRIFT

(11) **EP 0 697 463 B1**
(45) Veröffentlichungstag und Bekanntmachung des Hinweises auf die Patenterteilung: **29.05.2002**
(21) Anmeldenummer: 95112267.0
(22) Anmeldetag: 04.08.1995
(51) Int. Cl.: C12Q 1/56

(54) **Verfahren zur Reduzierung der Beeinflussung von diagnostischen Tests durch Heparin unter Verwendung von Metallsalzen und diagnostisches Reagenz für diagnostische Tests zur Bestimmung der Gerinnungsfähigkeit des Blutes enthaltend Metallsalze**
Process for the reduction of the influence of heparin in diagnostic tests comprising the use of metal salts and diagnostic reagent for diagnostic tests for the determination of blood coagulability comprising metal salts
Procédé de reduction d'influence d'héparine en essais diagnostiques comprenant l'utilisation de sels métalliques et réactif diagnostique pour essais diagnostiques de détermination de coagulabilité de sang contenant des sels métalliques

(30) Priorität: 20.08.1994 DE 4429660
(43) Veröffentlichungstag der Anmeldung: 21.02.1996
(73) Patentinhaber: Dade Behring Marburg GmbH, 35001 Marburg (DE)
(72) Erfinder: Fickenscher, Karl, Dr., D-35041 Marburg (DE); Zander, Norbert, Dr., D-35039 Marburg (DE)

(56) Entgegenhaltungen:
- CHEMICAL ABSTRACTS, Band 104, Nr. 15, 14. April 1986, Columbus, Ohio, USA A.M. CUMMING et al. "In vitro neutralization of heparin in plasma prior to the activated partial throm- boplastin time test: an assessment of four heparin antagonists and two anion exchange resins" Seite 340, Nr. 125 879r; & Thromb. Res. 1986, 41(1), 43-56
- CHEMICAL ABSTRACTS, Band 116, Nr. 19, 11. Mai 1992, Columbus, Ohio, USA Y. KAZAMA et al. "Modulation of protein C inhibitor activity by histi- dine-rich glycoprotein and platelet factor 4: role of zinc and calcium ions in the heparin-neutralizing ability of histidine-rich glyco- protein" Seite 42, Nr. 187 724t; & Thromb. Haemostasis 1992, 67(1), 50-5
- CHEMICAL ABSTRACTS, Band 115, Nr. 1, 8. Juli 1991, Columbus, Ohio, USA T. KOIDE et al. "Effects of zinc and calcium ions on the neutralization by histidine- -rich glycoprotein of the heparin-dependent activities of plasma proteinase inhibitors" Seite 565, Nr. 5 750x; & Int. Congr. Ser. - Excerpta Med. 1990, 873 (Protease Inhib.), 81-90
- CHEMICAL ABSTRACTS, Band 67, Nr. 23, 4. Dezember 1967, Columbus, Ohio, USA S.S. STIVALA et al. "Physicochemical studies of fractionated bovine heparin. IV. Copper(II) binding in relation to pH, molecular weight and biological activity" Seite 10861, Nr. 115 406n; & Arch. Biochem. Biophys. 122(1), 40-54 (1967)

## Beschreibung

In der vorliegenden Erfindung werden neue Möglichkeiten für diagnostische Tests und Reagenzien beschrieben, durch die die Beeinflussung durch Heparin modifiziert oder unterdrückt werden kann. Es werden neue Verfahren und Mittel zur Modifikation der Heparinempfindlichkeit von diagnostischen Tests beschrieben. Dabei wird entweder einem der diagnostischen Reagenzien oder dem Testansatz selbst wenigstens ein Metallsalz zugegeben, welches in der Lage ist, mit Heparin einen Komplex zu bilden und hierdurch dessen Aktivität verringert. Damit kann die Beeinflussung des Tests durch Heparin entweder völlig unterdrückt oder auf ein gewünschtes Maß eingestellt werden. Geeignete Metalle sind beispielsweise Zink oder Kupfer. In bestimmten Fällen kann auch eine Kombination mit bereits bekannten heparinneutralisierenden Stoffen, wie Polykationen, zu synergistischen Effekten führen und damit vorteilhaft sein.

Heparine sind polysulfatierte Mukopolysaccharide mit alternierenden Hexosamin- und Hexuronsäuregruppen unterschiedlicher Struktur. Sie werden aus der Mukosa und Lunge von Tieren isoliert. Die relativen Molekularmassen variieren je nach Quelle zwischen etwa 5 000 und 30 000.

Heparin kann an Antithrombin III ,Thrombin und Faktor Xa binden und dadurch die Reaktionsgeschwindigkeit zwischen der Protease und ihrem Inhibitor stark erhöhen.

Im Blutplasma bindet Heparin an Antithrombin III und macht aus diesem - langsam wirkenden - "Progressivinhibitor" somit einen "Sofortinhibitor-Komplex", der auf die Blutgerinnung wirksam ist.

In der Medizin wird Heparin häufig als therapeutische Substanz eingesetzt, mit der die Gerinnungsfähigkeit des Blutes vermindert wird. Dadurch kann der Entstehung von Thrombosen und Embolien vorgebeugt werden. Die Dosierung muß diagnostisch überwacht werden, um beispielsweise das Blutungsrisiko zu minimieren. Die dazu am häufigsten eingesetzten Tests, wie Thrombinzeit oder aktivierte partielle Thromboplastinzeit (aPTT), sind bei etwas höheren Dosierungen in der Regel zu empfindlich, so daß kein Gerinnungszeitpunkt mehr bestimmt werden kann.

Bei der Thrombinzeit handelt es sich um einen Kontrollparameter bei der Heparintherapie. Gemessen wird die Gerinnungszeit eines Citrat- oder Oxalatplasmas nach Zugabe einer genormten Menge Thrombin.

Es ist wünschenswert, die Heparinempfindlichkeit dieser Tests so einzustellen, daß sie einerseits im Bereich niedriger Heparinkonzentrationen ansprechen, andererseits jedoch im Bereich höherer Heparinkonzentrationen noch immer eine Auswertung erlauben.

Andere Teste (z.B. die Prothrombinzeit) zeigen mit Proben von unter Heparintherapie stehenden Patienten unerwünschte Störungen. In diesem Fall wäre eine vollständige Beseitigung des Heparineinflusses notwendig. Die Messung der Prothrombinzeit dient in erster Linie dazu, die Aktivität der Vitamin-K-abhängigen Gerinnungsfaktoren zu bestimmen. Bei hoher Heparindosierung wird eine verlängerte Gerinnungszeit gefunden und somit auf erniedrigte Plasmagehalte dieser Gerinnungsfaktoren geschlossen. Dadurch kann es zu einer unnötigen Gabe von Präparaten kommen, die diese Faktoren enthalten, so daß der Patient einer unnötigen und auch risikobehafteten Therapie ausgesetzt werden kann.

Um unerwünschte Einflüsse von Heparin auf diagnostische Tests auszuschließen, kann Heparin vor der Testdurchführung aus der Probe entfernt werden.

Es ist bekannt, daß Proben durch Behandlung mit Ionenaustauschern von Heparin befreit werden können. Im US Patent 5,000,854 beschreibt Yang ein Verfahren, bei dem aus Blut über an einen Träger gebundenes Protamin das Heparin entfernt wird. Das US Patent 4,199,502 (Babson und Turner, 1980) beschreibt ein Verfahren, bei der ein Komplex aus Protamin und Serumalbumin zur Probe gegeben wird und der nach einiger Zeit entstehende heparinhaltige Niederschlag anschließend abgefiltert wird.

Diese Verfahren sind sehr zeitaufwendig und beeinflussen die Proben negativ in Hinblick auf ihre Gerinnungseigenschaften.

Es ist ebenfalls möglich, das Heparin ohne Entfernung aus der Probe zu neutralisieren. Dazu werden der Probe Polykationen in genau dosierter Menge zugegeben.

Es bilden sich dann Komplexe aus den beiden gegensätzlich geladenen Makromolekülen. Durch die Neutralisation seiner negativen Ladungen verliert Heparin seine Wirksamkeit. Dies wurde bereits 1939 von Jorpes et al. (Lancet 2, 1939, 975-976) für Protamin beschrieben. Später wurden weitere geeignete Substanzen beschrieben, so z.B. Polybren ( Hexadimethrinbromid) von Godal (Scand.J.Clin. Lab. Invest. 12 (1960), 446-457).

Denkbar wäre auch die Zugabe von Polykationen zum Reagenz oder die separate Zugabe zum Testansatz. Diese Stoffe könnten dem Plasma, einem Reagenz oder separat dem Testansatz zugesetzt werden. Nachteilig ist allerdings, daß sie aufgrund ihrer polykationischen Natur häufig mit Bestandteilen der Reagenzien reagieren und so den Test massiv stören. So binden sie z.B. an Phospholipidoberflächen und neutralisieren deren gerinnungsfördernde Eigenschaften. Somit sind sie in phospholipidhaltigen Testsystemen, wie Prothrombinzeit oder aPTT, meist nicht einsetzbar. Nachteilig ist auch ihre Eigenschaft, Heparin so fest zu binden, daß es in seiner Wirkung völlig ausgeschaltet wird. Die Einstellung eines Testsystems auf eine reduzierte Heparinempfindlichkeit für eine Steuerung und Überwachung einer Therapie ist damit unmöglich.

Eine weitere Möglichkeit besteht darin, daß Heparin enzymatisch durch Zugabe des Enzyms Heparinase zur Probe abgebaut werden kann. Eine derartige Möglichkeit ist in der Literatur, bspw. WO 89/12692, mehrfach beschrieben. Diese Verfahren sind jedoch in der Regel zeitaufwendig, da die Spaltgeschwindigkeit des Enzyms relativ gering ist. Außerdem ist ein zusätzlicher Verfahrensschritt zum Heparinabbau notwendig. Die Einstellung eines Testsystems auf eine reduzierte Heparinempfindlichkeit für eine Steuerung und Überwachung einer Therapie ist auch in diesem Fall nicht möglich.

Es ist schon beschrieben, daß Heparin mit verschiedenen Metallionen Bindungen eingeht, z. B. Lages und Stivala, Biopolymers 12 (1973) 127-143. Die entstehenden Komplexe des Heparins mit Kalzium, Kupfer und Zink sind bisher vor allem in Reinsystemen biophysikalisch und biochemisch charakterisiert worden. Die Bindung kann zur Aufreinigung des Heparins verwendet werden; so wird z.B. in der WO 87/09347 ein affinitätschromatographischer Schritt über die Ausnutzung der Bildung eines Kupfer-Heparin-Komplexes beschrieben.

Es wurde aber auch schon beschrieben, daß Schwermetalle eine Reihe von enzymatischen Prozessen inhibieren. So ist die Inhibition der Faktor VII- Aktivität durch Zink eingehend untersucht worden (Pedersen et al., Thromb. Haemostasis 65(5); (1991); 528-534). Es erscheint dem Fachmann daher nicht sinnvoll, derartige Metallionen in diagnostische Tests oder Reagenzien, die der Bestimmung der Blutgerinnung dienen, die also im wesentlichen auch auf der Funktion von Enzymen beruhen, einzubringen.

Der vorliegenden Erfindung lag also das technische Problem zugrunde, eine Möglichkeit aufzuzeigen, die Heparinempfindlichkeit von an sich bekannten Gerinnungstests zu verringern bzw. auszuschalten.

Die Lösung dieses technischen Problems erfolgt durch die Bereitstellung der in den Patentansprüchen gekennzeichneten Ausführungsformen.

Überraschenderweise wurde aber im Rahmen der vorliegenden Erfindung gefunden, daß Metallsalze in geeigneter Konzentration dennoch eingesetzt werden können, um die Heparinempfindlichkeit diagnostischer Reagenzien zu modifizieren.

Im Rahmen der vorliegenden Erfindung wird ein Verfahren zur Reduzierung der Beeinflussung eines diagnostischen Tests durch Heparin gemäß den Ansprüchen 1-5 offenbart, bei dem mindestens ein Metallsalz, dessen Metallion mit Heparin einen Komplex bilden Kann, entweder in wenigstens ein Reagens des Testansatzes eingebracht wird oder aber dem Testansatz separat zugegeben wird.

Bei den diagnostischen Tests handelt es sich in bevorzugter Weise um Verfahren zur Bestimmung der Gerinnungsfähigkeit des Blutes.

Gegenstand der vorliegenden Erfindung sind auch diagnostische Reagenzien gemäß den Ansprüchen 6-8 zur Bestimmung der Thromboplastinzeit, der Trombinzeit oder der aktivierten partiellen Tromboplastinzeit die umfassen und zur Bestimmung der Trombinzeit wenigstens ein Metallsalz enthalten, dessen Metallion mit Heparin einen Komplex bilden kann, wodurch der störende Einfluß von Heparin auf den diagnostischen Test verhindert oder zumindest erheblich reduziert wird.

In bevorzugter Form sind die Metalle des Metallsalzes ausgewählt aus der Gruppe 1 oder der Gruppe 2 des Periodensystems der Elemente, wobei die Metalle Kupfer oder Zink besonders bevorzugt sind.

Die erfindungsgemäß verwendeten Metallsalze werden in einer Konzentration von größer 0 bis zu 20 mM Metallsalz eingesetzt. In bevorzugter Form bewegen sich die Konzentrationen der Metallsalze in einem Bereich von 50 bis 500 µM.

Es wurde auch gefunden, daß der überraschende, vorteilhafte Effekt, der durch die Lehre der vorliegenden Erfindung erzielt werden kann, weiter verbessert werden kann, wenn neben dem Metallsalz ein Polykation dem Zusatzmittel zugegeben wird. Ein hierbei besonders bevorzugtes Polykation ist das Polybren.

Die folgenden Beispiele dienen der Erläuterung der Erfindung. Sie belegen sowohl die weitgehende Ausschaltung des Heparineffekts auf einen Test (Beispiel 1), als auch synergistische Effekte mit anderen, bereits bekannten Heparinnneutralisatoren (Beispiel 1), als auch die Möglichkeit einer Einstellung der Heparinempfindlichkeit eines Testsystems auf einen gewünschten Grad (Beispiele 2 und 3).

### Beispiel 1

### Heparinneutralisierung durch Zugabe von Zinkchlorid und/oder Polybren zu einem Thromboplastinzeitreagenz

Ein Normalplasmapool wird mit Heparin (Liquemin(R), Roche) in Konzentrationen von 1 IU/ml bis 4 IU/ml aufgestockt. Ein Thromboplastinzeitreagenz (Thromborel R, Behringwerke AG) wird alternativ in:
a) Wasser
b) 100 µM Zinkchlorid
c) 100 µM Zinkchlorid, 10 mg/l Polybren
eingelöst.

Die Bestimmung der Thromboplastinzeit erfolgt nach den Vorschriften des Herstellers.

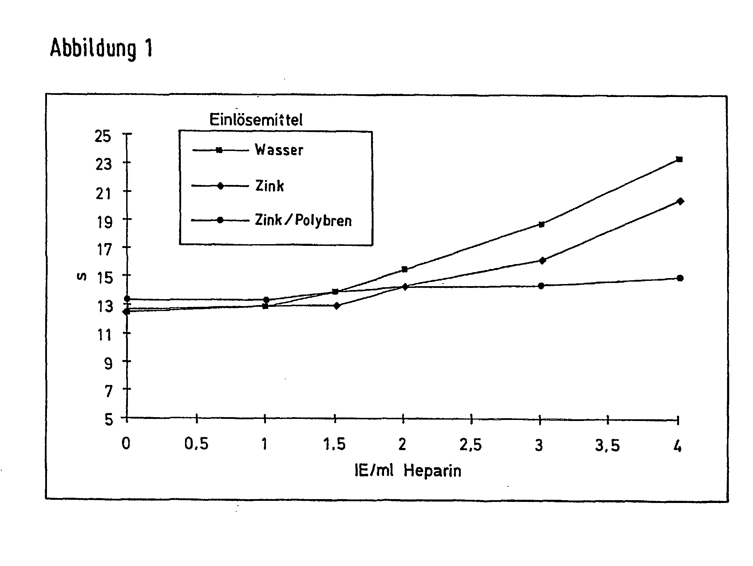

Abbildung 1 zeigt die Abhängigkeit der Prothrombinzeit von der Heparinkonzentration für die drei Einlösemittel des Prothrombinzeitreagenzes. Es ist klar ersichtlich, daß bei Verwendung der erfindungsgemäßen Zusatzmittel b) und c) der störende Einfluß von Heparin deutlich reduziert oder ganz ausgeschlossen werden kann.

### Beispiel 2

### Modifizierung der Heparinempfindlichkeit eines Thrombinzeitreagenzes durch Zink

Ein Frischplasma wurde mit Heparin (Liquemin, Roche) in Konzentrationen von 0.1 IU/ml bis 0.6 IU/ml aufgestockt. Ein Thrombinzeitreagenz (Test-Thrombin-Reagenz, Behringwerke AG) wurde in einer Konzentration von 3 IU/ml eingelöst. Der für dieses Reagenz vorgesehene Puffer wurde mit Zinkchlorid in Konzentrationen von 160 µM und 200 µM aufgestockt. Die Thrombinzeitbestimmung wurde wie folgt durchgeführt:
- 100 µl: Probe
- 100 µl: Puffer (ggf. mit Zink)
- 60 s: Inkubation bei 37°C
- 100 µl: Thrombin (3 IU/ml)
Messung des Gerinnungseintritts.

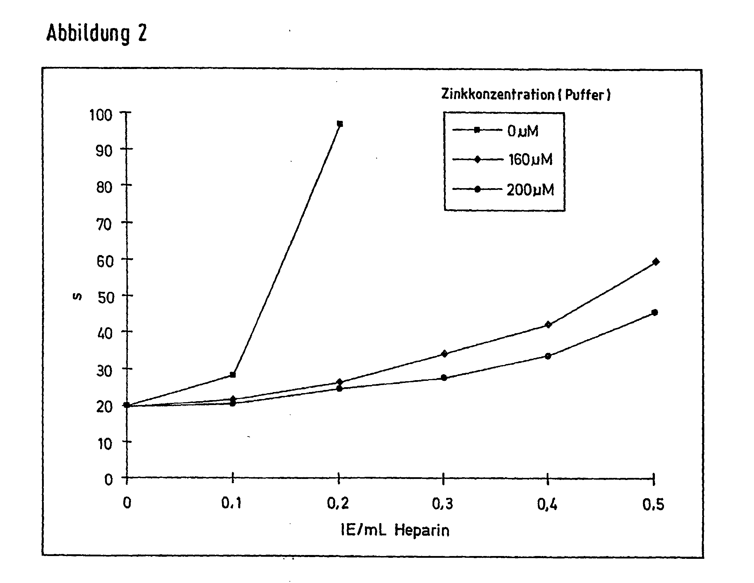

Abbildung 2 zeigt die gemessenen Thrombinzeiten in Abhängigkeit vom Heparingehalt der Probe und dem Zinkgehalt des Puffers. Gerade bei verhältnismäßig geringer Heparinkonzentration können die dadurch verursachten Störungen nahezu vollständig ausgeschlossen werden.

### Beispiel 3

### Modifizierung der Heparinempfindlichkeit der aktivierten partiellen Thromboplastinzeit durch Zink

Ein lyophilisierter menschlicher Normalplasmapool wurde mit Heparin (Liquemin, Roche) in Konzentrationen von 0.1 IU/ml bis 0.6 IU/ml aufgestockt. Die aPTT wurde mit Pathromtin (Behringwerke AG) entsprechend den Angaben des Herstellers durchgeführt. Wahlweise wurde entweder dem Aktivatorreagenz oder dem Startreagenz (Calciumchlorid) Zinkchlorid in Konzentrationen von 50 µM und 100 µM zugesetzt.

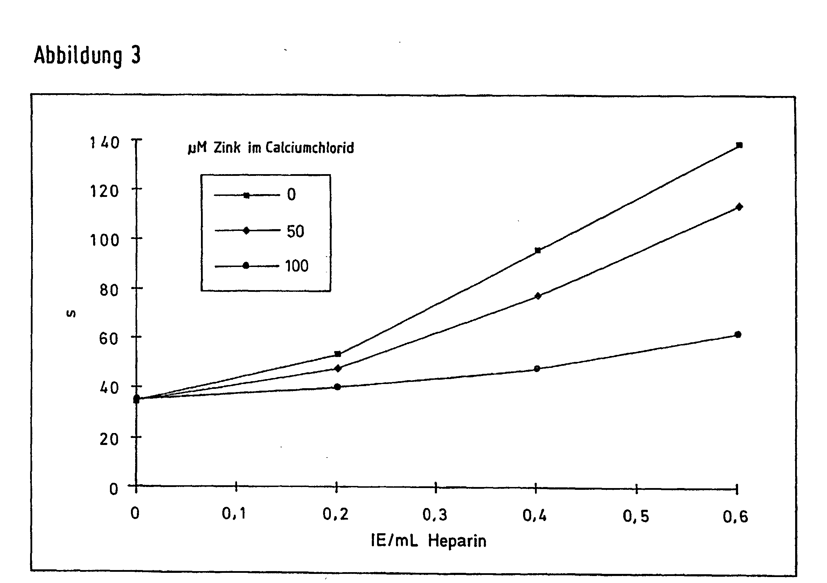

Abbildung 3 zeigt die gemessenen aPTTs in Abhängigkeit vom Heparingehalt der Probe und dem Zinkgehalt des Startreagenzes. Für eine Zugabe des Zinkchlorids zum Aktivatorreagenz ergaben sich ähnliche Meßwerte.

Die Abbildungen zeigen:

### Abbildung 1 : Heparinneutralisierung durch Zugabe von Zinkchlorid und/oder Polybren zu einem Thromboplastinzeitreagenz

Thromborel S wurde wie in Beispiel 1 beschrieben in verschiedenen Lösemitteln eingelöst. Ein Normalplasmapool wurde mit Heparin (Liquemin(R), Roche) in Konzentrationen von 1 IU/ml bis 4 IU/ml aufgestockt.

Abbildung 1 zeigt die Abhängigkeit der Prothrombinzeit von der Heparinkonzentration für die drei Einlösemittel des Prothrombinreagenzes.

### Abbildung 2 : Modifizierung der Heparinempfindlichkeit eines Thrombinzeitreagenzes durch Zink

Das Test-Thrombin-Reagenz wurde wie in Beispiel 2 beschrieben eingelöst. Ein Frischplasma wurde mit Heparin (Liquemin, Roche) in Konzentrationen von 0.1 IU/ml bis 0.6 IU/ml aufgestockt.

Die Abbildung zeigt die gemessenen Thrombinzeiten in Abhängigkeit vom Heparingehalt der Probe und dem Zinkgehalt des Puffers.

### Abbildung 3 : Modifizierung der Heparinempfindlichkeit der aktivierten partiellen Thromboplastinzeit durch Zink

Pathromtin wurde wie in Beispiel 3 beschrieben vorbereitet. Ein lyophilisierter menschlicher Normalplasmapool wurde mit Heparin (Liquemin, Roche) in Konzentrationen von 0.1 IU/ml bis 0.6 IU/ml aufgestockt.

Abbildung 3 zeigt die gemessenen aPTTs in Abhängigkeit vom Heparingehalt der Probe und dem Zinkgehalt des Startreagenzes. Für eine Zugabe des Zinkchlorids zum Aktivatorreagenz ergaben sich ähnliche Meßwerte.

Die gemessene Zeit ist bei allen drei Abbildungen in Sekunden (s) angegeben.

## Patentansprüche

1. Verfahren zur Verminderung der Störung eines diagnostischen Verfahrens durch Heparin, **dadurch gekennzeichnet, daß** wenigstens ein Metallsalz aus der Gruppe der Kupfer- und Zinksalze in wenigstens ein Reagens des Testansatzes eingebracht oder dem Testansatz separat zugegeben wird und daß das Metallsalz in einer Konzentration von größer 0 bis zu 20 mM eingesetzt wird.

2. Verfahren nach Anspruch 1, **dadurch gekennzeichnet, daß** das Metallsalz in einer Konzentration von 50 bis 500 µM eingesetzt wird.

3. Verfahren gemäß Anspruch 1 oder 2, wobei das diagnostische Verfahren ein Verfahren zur Bestimmung eines Parameters der Gerinnungsfähigkeit des Blutes ist.

4. Verfahren gemäß Anspruch 3, wobei das diagnostische Verfahren die Bestimmung der Thrombinzeit, der Thromboplastinzeit, oder der aktivierten partiellen Thromboplastinzeit ist.

5. Verfahren nach mindestens einem der Ansprüche 1-4, wobei mindestens ein Reagenz Polybren enthält.

6. Diagnostisches Reagenz zur Bestimmung der Thromboplastinzeit, der Thrombinzeit oder der aktivierten partiellen Thromboplastinzeit, **dadurch gekennzeichnet, daß** es wenigstens ein Metallsalz aus der Gruppe der Zink- oder Kupfersalze enthält, wobei dieses Metallsalz in einer Konzentration von größer 0 bis zu 20 mM eingesetzt wird.

7. Diagnostisches Reagenz nach Anspruch 6, **dadurch gekennzeichnet, daß** das Metallsalz in einer Konzentration von 50 bis 500 µM eingesetzt wird.

8. Diagnostisches Reagenz nach Anspruch 6 oder 7, **dadurch gekennzeichnet, daß** es zusätzlich Polybren enthält.

## Claims

1. Method for reducing interference in a diagnostic method by heparin, **characterized in that** at least one metal salt from the group consisting of copper and zinc salts is introduced into at least one reagent of the test batch or is added separately to the test batch and **in that** the metal salt is employed in a concentration of greater than 0 to 20 mM.

2. Method according to Claim 1, **characterized in that** the metal salt is employed in a concentration of 50 to 500 µM.

3. Method according to Claim 1 or 2, wherein the diagnostic method is a method for the determination of a parameter of the coagulability of blood.

4. Method according to Claim 3, wherein the diagnostic method is the determination of the thrombin time, the thromboplastin time or the activated partial thromboplastin time.

5. Method according to at least one of claims 1-4, wherein at least one reagent comprises Polybrene.

6. Diagnostic reagent for determination of the thromboplastin time, the thrombin time or the activated partial thromboplastin time, **characterized in that** it comprises at least one metal salt from the group consisting of zinc or copper salts, this metal salt being employed in a concentration of greater than 0 to 20 mM.

7. Diagnostic reagent according to Claim 6, **characterized in that** the metal salt is employed in a concentration of 50 to 500 µM.

8. Diagnostic reagent according to Claim 6 or 7, **characterized in that** it additionally comprises Polybrene.

## Revendications

1. Procédé pour éviter la perturbation par l'héparine d'un procédé de diagnostic, **caractérisé en ce qu'**on ajoute séparément au mélange d'essai ou introduit dans au moins un réactif du mélange d'essai au moins un sel métallique choisi dans le groupe des sels de cuivre et de zinc, et **en ce que** le sel métallique est utilisé à une concentration de > 0 à 20 mM.

2. Procédé selon la revendication 1, **caractérisé en ce que** le sel métallique est utilisé à une concentration de 50 à 500 µM.

3. Procédé selon la revendication 1 où 2, dans lequel le procédé de diagnostic est un procédé pour la détermination d'un paramètre du pouvoir de coagulation du sang.

4. Procédé selon la revendication 3, dans lequel le procédé de diagnostic est la détermination du temps de thrombine, du temps de céphaline ou du temps de céphaline-kaolin.

5. Procédé selon au moins l'une des revendications 1 à 4, dans lequel au moins un réactif contient du Polybrene.

6. Réactif de diagnostic pour la détermination du temps de céphaline, du temps de thrombine ou du temps de céphaline-kaolin, **caractérisé en ce qu'**il contient au moins un sel métallique choisi dans le groupe des sels de cuivre ou de zinc, ce sel métallique étant utilisé à une concentration de > 0 à 20 mM.

7. Réactif de diagnostic selon la revendication 6, **caractérisé en ce que** le sel métallique est utilisé à une concentration de 50 à 500 µM.

8. Réactif de diagnostic selon la revendication 6 ou 7, **caractérisé en ce qu'**il contient en outre du Polybrene.
